# EUROPÄISCHE PATENTSCHRIFT

(11) **EP 1 754 518 B1**
(45) Veröffentlichungstag und Bekanntmachung des Hinweises auf die Patenterteilung: **25.02.2009**
(21) Anmeldenummer: 06016395.3
(22) Anmeldetag: 05.08.2006
(51) Int. Cl.: A61Q 19/00, A61Q 19/10, A61K 8/20, A61K 8/34

(54) **Selbsterwärmende Hautreinigungszusammensetzung**
Self-heating skin cleansing composition
Composition auto-chauffante pour le nettoyage de la peau

(30) Priorität: 17.08.2005 DE 102005039166
(43) Veröffentlichungstag der Anmeldung: 21.02.2007
(73) Patentinhaber: Henkel AG & Co. KGaA, 40589 Düsseldorf (DE)
(72) Erfinder: Cortekar, Hans-Wolfgang, 42855 Remscheid (DE)

(56) Entgegenhaltungen:
- DE-A1- 10 009 252
- DE-A1-1102004 038 77
- DE-U1- 9 211 006

## Beschreibung

Die Erfindung betrifft eine selbsterwärmende Hautreinigungszusammensetzung mit verbesserter Lager- und Temperaturstabilität zur gründlichen und schonenden Reinigung der Haut, die bei der Anwendung mit Wasser vermischt wird und dabei Wärme freisetzt. Durch die freigesetzte Wärme kommt es zu einem sensorisch angenehmen Hauteindruck, einer Verbesserung des Reinigungseffektes, zu einer erhöhten Freisetzung eventuell enthaltener Duftstoffe und zu einer verbesserten Wirkung eventuell enthaltener hautkosmetischer Wirkstoffe.

Die Verwendung von wasserfreien Salzen mit negativer Lösungsenthalpie, die beim Lösen in Wasser Hydratationswärme freisetzen, zur Herstellung von kosmetischen Zubereitungen, die sich bei der Anwendung erwärmen, ist im Stand der Technik bereits bekannt. DE 2317140 C2 offenbart wässrige Haut- und Haarbehandlungsmittel, die kurz vor der Anwendung durch Hinzufügen von Calciumchlorid oder Magnesiumsulfat erwärmt werden. In DE 19624870 A1 und WO 97/02802 A2 sind Zahnpflegemittel beschrieben, die bei Zutritt von Wasser oder Speichel bei der Zahnreinigung Wärme freisetzen. In US 3,250,680 und WO 93/08793 A1 sind Hautreinigungsmittel beschrieben, die z. B. als Gesichtsmaske angewendet werden können und als Wärme freisetzende Komponente ein entwässertes Molekularsieb, das heißt einen Zeolith, in einem wasserfreien Träger enthalten. US 4,159,316 offenbart wasserfreie Zeolith-haltige Zahnpasten, in denen der Zeolith auch als Abrasivkomponente dient. US 5,747,004 offenbart Zahnpasten, die als Hydratationswärme freisetzendes Salz wasserfreies Natriumcarbonat enthalten, das in Wasser hydrolysiert und einen stark basischen pH-Wert einstellt (pH 9,36 - 10,7). Derart hohe pH-Werte sind für die Anwendung auf der Haut, insbesondere der Gesichtshaut, weniger geeignet und für empfindliche Haut sogar ungeeignet. EP 1 186 286 A1 offenbart selbsterwärmende Hautreinigungsmittel mit Partikeln aus Natriumpolyacrylat von 1 - 80 µm Durchmesser, die sich bei der Anwendung mit Wasser nach und nach auflösen. In WO 01/12150 A1 findet sich der Hinweis auf eine selbsterwärmende Gesichtsmaske der Marke Bioré, die Zeolith (sodium silicoaluminate) und verkapselte Ultramarinpigmente enthält und frei ist von wasserlöslichen Salzen, die sich in Wasser mit negativer Lösungsenthalpie lösen. Beim Verreiben der Maskenzusammensetzung auf der Haut werden diese Pigmente durch die Zerstörung der Verkapselung freigesetzt und zeigen durch das Verfärben der Zusammensetzung das Ende des Reinigungsvorgangs an. WO 01164176 A1 offenbart selbsterwärmende Hautreinigungszusammensetzungen, die wasserfreie Tenside enthalten, die bei Raumtemperatur als festes Pulver vorliegen, insbesondere Natriumkokosacylisethionat, Kokosacylamidopropyl-Betain und Dinatriumsulfobernsteinsäure-mono(C₁₂₋₁₈)-alkylester. DE 9211006 U offenbart ein Haarbehandlungsmittel in Form eines Kits aus einer Zusammensetzung (A), enthaltend ein Wärme entwickelndes Salz und ein Verdickungsmittel, und einer glycolischen Tensidlösung/dispersion (B), die bis zum Gebrauch getrennt voneinander gelagert werden.

Es wurde nun festgestellt, dass die bekannten Zubereitungen entweder zur Anwendung als Hautreinigungsmittel weniger geeignet sind, da sie nicht genügend Schaum entwickeln und keine befriedigende Reinigung auch stärker verschmutzter Haut erzielen, oder aber eine zur Dosierung als flüssige oder pastenförmige Zubereitungen vom Typ einer Flüssigseife oder einer Waschpaste ungeeignete Konsistenz aufweisen. Als Nachteil der bekannten Zusammensetzungen, die pulverförmige Tenside enthalten und eine bei Raumtemperatur gut dosierbare Konsistenz aufweisen, hat sich überraschenderweise ihre mangelnde Lagerstabilität bei höheren Temperaturen, insbesondere bei Temperaturen von 40 °C und darüber, herausgestellt.

Eine Aufgabe der vorliegenden Erfindung war es, ein Hautreinigungsmittel zu entwickeln, das sich wie eine Flüssigseife oder eine Handwaschpaste anwenden lässt. Eine weitere Aufgabe der vorliegenden Erfindung war es, ein Hautreinigungsmittel zu entwickeln, das einen voluminösen Schaum und gleichzeitig so viel Wärme entwickelt, dass ein sensorisch angenehmes Hautgefühl hervorgerufen wird. Eine weitere Aufgabe der vorliegenden Erfindung war es, ein Hautreinigungsmittel zu entwickeln, mit dem eine potentiefe, aber dennoch schonende Reinigung der Haut erreicht wird. Eine weitere Aufgabe der vorliegenden Erfindung war es, ein Hautreinigungsmittel mit verbesserter Lagerstabilität auch bei höheren Temperaturen, insbesondere bei Temperaturen von 40 °C und darüber, zu entwickeln.

Gegenstand der vorliegenden Erfindung ist eine wasserfreie, selbsterwärmende Hautreinigungszusammensetzung, die beim Vermischen mit Wasser Hydratationswärme erzeugt, enthaltend (jeweils bezogen auf die Gesamtzusammensetzung)
a) mindestens 15 Gew.-% mindestens eines wasserlöslichen mehrwertigen C₂ - C₉-Alkanols mit 2 - 6 Hydroxylgruppen und/oder mindestens eines wasserlöslichen Polyethylenglycols mit 3 - 20 Ethylenoxid-Einheiten und/oder mindestens eines wasserlöslichen Polypropylenglycols und/oder mindestens eines wasserlöslichen Anlagerungsproduktes von Ethylenoxid an Propylenglycol oder an Polypropylenglycol sowie Mischungen dieser Komponenten,
b) mindestens 5 Gew.-% mindestens eines anionischen, zwitterionischen, ampholytischen, nichtionischen oder kationischen Tensids oder Mischungen hiervon,
c) mindestens 5 Gew.% mindestens eines wasserlöslichen Salzes, das sich in Wasser mit negativer Lösungsenthalpie löst sowie
d) mindestens 0,1 Gew.-% mindestens eines organischen und/oder anorganischen Verdickungsmittels,
wobei mindestens 50 Gew.-%, bevorzugt mindestens 80 Gew.-% und besonders bevorzugt 100 Gew.-% der enthaltenen Tenside bei 20°C in flüssiger oder, bevorzugt, pastöser, fließfähiger Form vorliegen (Anteilsangaben bezogen auf das Gesamtgewicht der enthaltenen Tenside) und die Zusammensetzung frei von Molekularsieben oder Zeolithen ist.

Als wasserfrei wird dabei erfindungsgemäß eine Zusammensetzung angesehen, die maximal 2 Gew.-% Wasser, bevorzugt maximal 1 Gew.-% und besonders bevorzugt maximal 0,5 Gew.% Wasser enthält

Die erfindungsgemäßen Hautreinigungszusammensetzungen enthalten mindestens ein wasserlösliches Polyol, ausgewählt aus wasserlöslichen mehrwertigen C₂ -C₈-Alkanolen mit 2 - 6 Hydroxylgruppen, wasserlöslichen Polyethylenglycolen mit 3 - 20 Ethylenoxid-Einheiten, wasserlöslichen Polypropylenglycolen und wasserlöslichen Anlagerungsprodukten von Ethylenoxid an Propylenglycol oder an Polypropylenglycol sowie Mischungen dieser Komponenten. Unter Wasserlöslichkeit versteht man erfindungsgemäß, dass sich wenigstens 5 Gew.-% des Polyols bei 20 °C klar lösen oder aber - im Falle langkettiger oder polymerer Polyole - durch Erwärmen der Lösung auf 50 °C bis 60 °C in Lösung gebracht werden können. In einer bevorzugten Ausführungsform der Erfindung sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Bevorzugte wasserlösliche Polyethylenglycole sind ausgewählt aus PEG-3. PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose können erfindungsgemäß bevorzugt sein.

In einer weiteren bevorzugten Ausführungsform der Erfindung können einwertige C₂-C₆-Alkohole wie Ethanol, Isopropanol oder Ethoxydiglycol in einer Gesamtmenge bis 5 Gew.-% enthalten sein. Beim Einsatz dieser einwertigen C₂-C₆-Alkohole ist zu beachten, dass die oben definierte Bedingung "wasserfrei" für die gesamte erfindungsgemäße Zusammensetzung erfüllt bleibt.

Die erfindungsgemäßen Zusammensetzungen enthalten mindestens ein wasserlösliches Polyol in einer Gesamtmenge von mindestens 15 Gew.-%. Bevorzugte Ausführungsformen der Erfindung sind dadurch gekennzeichnet, dass das/die wasserlösliche/n Polyol/e in einer Gesamtmenge von mindestens 20 Gew.-%, bevorzugt 30 - 70 Gew.-%, besonders bevorzugt 40 - 60 Gew.-% und außerordentlich bevorzugt 45 - 55 Gew.-%, bezogen auf die Gesamtzusammensetzung, enthalten ist/sind. Durch die Art und Menge der wasserlöslichen Polyole lässt sich in gewissem Maß die Konsistenz sowie das Löse- und Dispergierverhalten des Trägers beeinflussen. Darüber hinaus haben einige dieser wasserlöslichen Polyole ebenfalls eine negative Mischungsenthalpie mit Wasser, d. h. beim Mischen mit Wasser wird Wärme freigesetzt. Aus diesem Grund ist es besonders bevorzugt, Mischungen von zwei oder mehr unterschiedlichen wasserlöslichen Polyolen einzusetzen, um ein optimales Anwendungsprofil zu erhalten. Gemische von 1,2-Propylenglycol, Polyethylenglycol, insbesondere PEG-8, und Glycerin und gewünschtenfalls Sorbit sind außerordentlich bevorzugte Träger.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für bevorzugte anionische Tenside sind, jeweils in Form der ihrer Salze,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare

Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der

Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate,
- Alkylpolyglycolethersulfate der Formel R-O(CH₂-CH₂O)ₓ-OSO₃H, in der R eine bevorzugt lineare Alkylgruppe mit 8 bis 30 C-Atomen und x = 0 oder 1 - 12, bevorzugt 1 - 4, ist,
- Ester der Weinsäure und Zitronensäure mit Alkoholen, die Anlagerungsprodukte von etwa 2-15 Ethylenoxid und/oder Propylenoxid-Einheiten an Fettalkohole mit 8 bis 22 C-Atomen darstellen, insbesondere Citronensäurealkylpolyglycolethermonoester, Citronensäurealkylpolyglycoletherdiester, Weinsäurealkylpolyglycolethermonoester und Weinsäurealkylpolyglycoletherdiester, besonders bevorzugt solche, bei denen der Alkylrest von linearen Fettalkoholen mit 8 bis 18, bevorzugt 12 C-Atomen, stammt und durchschnittlich 5 - 10, bevorzugt durchschnittlich 7 - 9 Ethylenoxid-Einheiten pro Molekül enthalten sind,
- Alkyl- und/oder Alkenyletherphosphate der Formel (II),

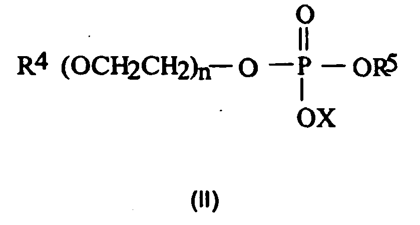

in der R⁴ bevorzugt für einen aliphatischen Kohlenwasserstoffrest mit 8 bis 30 Kohlenstoffatomen, R⁵ für Wasserstoff, einen Rest (CH₂CH₂O)ₙR¹⁸ oder X, n für Zahlen von 1 bis 10 und X für Wasserstoff, ein Alkali- oder Erdalkalimetall oder NR⁶R⁷R⁸R⁹, mit R⁶ bis R⁹ unabhängig voneinander stehend für Wasserstoff oder einen C₁ - C₄ - Kohlenwasserstoffrest, steht,
- sulfatierte Fettsäurealkylenglycolester der Formel R¹⁰CO(A)kO)ₙSO₃M, in der R¹⁰CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV) 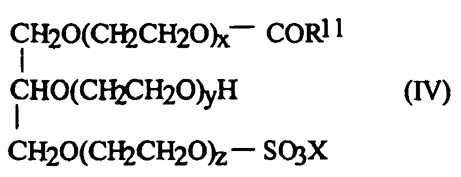 in der R¹¹CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht,
- Amidethercarbonsäuren gemäß EP 0 690 044,
- Kondensationsprodukte aus C₈ - C₃₀ - Fettalkoholen mit Proteinhydrolysaten und/oder Aminosäuren und deren Derivaten, sogenannte Eiweißfettsäurekondensate, z. B. Lamepon^{®}, Gluadin^{®}, Hostapon^{®} KCG oder Amisoft^{®}.

Die Salze dieser Tenside sind bevorzugt ausgewählt aus den Natrium-, Kalium- und Ammonium- sowie den Mono-, Di- und Trialkanolammoniumsalzen mit 2 bis 4 C-Atomen in der Alkanolgruppe. Bei der Auswahl der Salze für die erfindungsgemäßen Zusammensetzungen ist zu beachten, dass mindestens eines der Tenside bei 20°C in flüssiger oder pastöser, fließfähiger Form vorliegt. In der Regel sind die Natriumsalze der genannten Aniontenside bei 20°C fest, wohingegen die Mono-, Di- und Trialkanolammoniumsalze mit 2 bis 4 C-Atomen in der Alkanolgruppe in der Regel bei 20°C flüssig oder in pastöser, fließfähiger Form vorliegen. Ein bevorzugtes Tensid dieser Art ist ein Tensidgemisch von Alkylpolyglycolethersulfaten der Formel R-O(CH₂-CH₂O)ₓ-OSO₃H, in der R eine lineare Alkylgruppe mit 12 oder 14 C-Atomen und x = 2 ist und als Monoisopropanolammoniumsalz vorliegt. Ein derartiges Tensid ist in wasserfreier Form beispielsweise unter dem Handelsnamen Marlinat 242/90 M erhältlich.
Bei den Tensiden, die bei 20°C in flüssiger Form vorliegen, liegt der Schmelzpunkt unter 20°C, so dass die Tenside bei 20°C komplett durchgeschmolzen und flüssig sind. Bei den Tensiden, die bei 20°C in pastöser, fließfähiger Form vorliegen, liegt der Schmelz- oder Erweichungspunkt im Bereich von 20 - 35 °C, so dass die Tenside bei 20°C zwar noch nicht komplett durchgeschmolzen und flüssig sind, wohl aber eine fließfähige, pastöse Konsistenz aufweisen. Es wurde überraschend festgestellt, dass die Verwendung von Tensiden, die bei 20°C in flüssiger oder pastöser, fließfähiger Form vorliegen, die Temperatur- und Lagerstabilität auch bei höheren Temperaturen, insbesondere bei 40 °C und darüber, gegenüber dem Stand der Technik deutlich verbessert. Insbesondere konnte beobachtet werden, dass bei Verwendung von Tensiden, die bei 20°C in pastöser, fließfähiger Form vorliegen, eine günstige Konsistenz der erfindungsgemäßen Zusammensetzungen bei 20°C erhalten wurde, die auch bei Lagerung bei 40 °C und darüber stabil blieb und sich das Produkt weder verdickte noch zu flüssig wurde. Besonders bevorzugte erfindungsgemäße Zusammensetzungen sind daher dadurch gekennzeichnet, dass mindestens 50 Gew.-%, bevorzugt mindestens 80 Gew.-% und besonders bevorzugt 100 Gew.-% der enthaltenen Tenside bei 20°C in flüssiger oder, bevorzugt, pastöser, fließfähiger Form vorliegen (Anteilsangaben bezogen auf das Gesamtgewicht der enthaltenen Tenside).

Als zwitterionische Tenside werden solche oberflächenaktiven Verbindungen bezeichnet, die im Molekül mindestens eine quartäre Ammoniumgruppe und mindestens eine -COO⁻- oder -SO₃⁻ -Gruppe tragen. Besonders bevorzugte zwitterionische Tenside sind die sogenannten Betain-Tenside wie die N-Alkyl-N,N-dimethylammonium-glycinate, z. B. das Kokosalkyl-dimethylammoniumglycinat, N-Acyl-aminopropyl-N,N-dimethylammoniumglycinate, z. B. das Kokosacylaminopropyldimethylammoniumglycinat, das C₁₂-C₁₈-Alkyl-dimethyl-acetobetain, das Kokosamidopropyl-dimethyl-acetobetain, 2-Alkyl-3-carboxymethyl-3-hydroxyethyl-imidazoline und Sulfobetaine mit jeweils 8 bis 18 C-Atomen in der Alkyl- oder Acylgruppe sowie das Kokosacylaminoethylhydroxyethylcarboxymethylglycinat. Ein besonders bevorzugtes zwitterionisches Tensid ist das unter der INCI-Bezeichnung Cocamidopropyl Betaine bekannte N,N-Dimethyl-N-(lauroylamidopropyl)ammoniumacetobetain.
Die Betain-Tenside zählen zu den schäumenden Tensiden.

Unter ampholytischen Tensiden werden solche oberflächenaktiven Verbindungen verstanden, die außer einer C₈ - C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Erfindungsgemäß bevorzugte ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂- C₁₈-Acylsarcosin.

Unter nichtionischen Tensiden werden solche Tenside verstanden, die eine lipophile, bevorzugt lineare Alkyl- oder Acylgruppe mit 8-22 C-Atomen und als hydrophile Gruppe einen Glucosid- oder Polyglucosidrest, einen Glycerin- oder Polyglycerinrest, einen Sorbitanrest oder einen Polyglycoletherrest oder mehrere dieser Reste enthalten. Erfindungsgemäß bevorzugte nichtionische Tenside sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, wie beispielsweise die unter der Verkaufsbezeichnung Dehydol^{®} LT (Cognis) erhältlichen Typen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter der Verkaufsbezeichnung Dehydol^{®} LS (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxylierte Triglyceride,
- alkoxylierte Fettsäurealkylester der Formel R¹²CO-(OCH₂CHR¹³)_{w}OR¹⁴, in der R¹²CO für einen linearen oder verzweigten, gesättigten und/oder ungesättigten Acylrest mit 6 bis 22 Kohlenstoffatomen, R¹³ für Wasserstoff oder Methyl, R¹⁴ für lineare oder verzweigte Alkylreste mit 1 bis 4 Kohlenstoffatomen und w für Zahlen von 1 bis 20 steht,
- Aminoxide, z. B. N,N-Dimethyl-N-kokosalkylaminoxid,
- Amidoalkylaminoxide, z. B. Cocamidopropyldimethylaminoxid,
- Hydroxymischether, beispielsweise gemäß DE-OS 19738866,
- Sorbitanfettsäureester und Anlagerungsprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglycoside gemäß Formel R¹⁵O-[G]p, in der R¹⁵ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Die Alkyl- und Alkenyloligoglycoside können sich von Aldosen bzw. Ketosen mit 5 oder 6 Kohlenstoffatomen, vorzugsweise von Glucose, ableiten. Vorzugsweise werden Alkyl- und/oder Alkenyloligoglycoside mit einem mittleren Oligomerisierungsgrad p von 1,1 bis 3,0, bevorzugt kleiner als 1,7 insbesondere 1,2-1,4 eingesetzt.
- Zuckertenside vom Typ der Fettsäure-N-alkylpolyhydroxyalkylamide der Formel R¹⁶CO-NR¹⁷Z, in der R¹⁶CO für einen aliphatischen Acylrest mit 6 bis 22 Kohlenstoffatomen, R¹⁷ für Wasserstoff, einen Alkyl- oder Hydroxyalkylrest mit 1 bis 4 Kohlenstoffatomen und Z für einen linearen oder verzweigten Polyhydroxyalkylrest mit 3 bis 12 Kohlenstoffatomen und 3 bis 10 Hydroxylgruppen steht.

Als nichtionische Tenside sind nur solche geeignet, die in wasserfreier Form zur Verfügung stehen. Aufgrund ihrer schäumenden und/oder hautverträglichen Eigenschaften besonders bevorzugt sind die Anlagerungsprodukte von Ethylenoxid an Fettalkohole, an Fettsäuren, an Fettsäuremono- oder diglyceride, an Fettsäurealkanolamide, an Methylglucosidfettsäureester oder an Alkyl- und Alkenyloligoglycoside sowie die Alkyl- und Alkenyloligoglycoside selbst, weiterhin Aminoxide und Amidoalkylaminoxide, wobei die Fettketten lineare oder verzweigte Alkyl- oder Alkenylgruppen mit 5 oder 6 bis 30, bevorzugt 7 oder 8 bis 22 und besonders bevorzugt 11 oder 12 bis 17 oder 18 Kohlenstoffatomen darstellen. Besonders bevorzugt sind die Anlagerungsprodukte von Ethylenoxid an C₁₂―C₁₈-Fettalkohole, die einen durchschnittlichen Ethoxylierungsgrad von 7 - 10 aufweisen, insbesondere Laureth-4, Laureth-7 und Laureth-10 sowie Mischungen dieser Substanzen mit entsprechend ethoxylierten C₁₂ ―C₁₈-Fettalkoholen, insbesondere mit ethoxyliertem Myristylalkohol, ethoxyliertem Palmitylalkohol, ethoxyliertem Cetylalkohol und ethoxyliertem Stearylalkohol. Diese Substanzen liegen bei 20°C in flüssiger oder pastöser, fließfähiger Form vor. Entsprechende bevorzugte Handelsprodukte sind beispielsweise Dehydol 100 oder Dehydol LT 7 von Cognis. Weiterhin bevorzugt sind die Alkyl- und Alkenyloligoglycoside, besonders bevorzugt die Alkyloligoglucoside, insbesondere C₁₀-C₁₆- Alkylglucoside mit einer Mischung von Alkylglucosiden mit Alkylkettenlängen mit 10 - 16 Kohlenstoffatomen.

Eine weitere besonders geeignete Gruppe nichtionischer Tenside sind die Poly-(C₂-C₃)-alkylenglycol-modifizierten Silicone, deren frühere INCl-Bezeichnung Dimethicone Copolyol lautete, mit den aktuellen INCl-Bezeichnungen PEG-x Dimethicone (mit x = 2 - 20, bevorzugt 3-17. besonders bevorzugt 11-12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3-30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14-18 und besonders bevorzugt 16 stehen), die z. B. unter den Handelsbezeichnungen Dow Corning 193 (PEG-12 Dimethicone von Dow Corning) oder Abil Care 85 (Bis-PEG/PPG-16/16 PEG/PPG 16/16 Dimethicone von Goldschmidt) im Handel sind.

Bevorzugte erfindungsgemäße Hautreinigungszusammensetzung sind dadurch gekennzeichnet, dass das mindestens eine wasserfreie Tensid, das bei 20°C in flüssiger oder pastöser, fließfähiger Form vorliegt, ausgewählt ist aus Alkylpolyglycolathersulfaten der Formel R-O(CH₂-CH₂O)ₓ-OSO₃H, in der R eine bevorzugt lineare Alkylgruppe mit 8 bis 30 C-Atomen und x = 0 oder 1-12, bevorzugt 1 - 4, ist, Citronensäurealkylpolyglycolethermonoestern, Citronensäurealkylpolyglycoletherdiestern, Weinsäurealkylpolyglycolethermonoestern, Weinsäurealkylpolyglycoletherdiestern, Alkylsulfaten, Alkansulfonaten, Alpha-Olefinsulfonaten, Acylisethionaten, Acyltauriden, Acylsarkosiden, Sulfobemsteinsäuremonoalkylester-Salzen und Alkylpolyglycolethercarboxylaten in Form ihrer Alkalimetallsalze, Ammoniumsalze oder Mono-, Di- oder/und Trialkanolammoniumsalze, besonders bevorzugt in Form ihrer Mono-, Di- oder/und Trialkanolammoniumsalze, außerordentlich bevorzugt in Form ihrer Monoalkanolammoniumsalze, insbesondere in Form ihrer Monoisoalkanolammoniumsalze, wie z. B. die Monoisopropanolammoniumsalze oder Monoisobutanolammoniumsalze, weiterhin aus Alkyl- oder Alkenyloligoglucosiden mit einer durchschnittlichen Alkyl- oder Alkenylkettenlänge von 8 - 12 Kohlenstoffatomen, Anlagerungsprodukten von 1-10 Ethylenoxideinheiten an gesättigte, lineare und verzweigte Alkanole mit 8 - 20 Kohlenstoffatomen, sowie Mischungen der vorgenannten Substanzen.
Besonders bevorzugte erfindungsgemäße Zusammensetzungen sind dadurch gekennzeichnet, dass sie mindestens 5 Gew.-% einer Tensidmischung aus Alkyl- oder Alkenyl-oligoglucosiden mit einer durchschnittlichen Alkyl- oder Alkenylkettenlänge von 8 - 12 Kohlenstoffatomen und Anlagerungsprodukten von 1 - 10 Ethylenoxideinheiten an gesättigte, lineare und verzweigte Alkanole mit 8 - 20 Kohlenstoffatomen enthalten.

Erfindungsgemäß besonders bevorzugte Zusammensetzungen sind dadurch gekennzeichnet, dass mindestens 60 Gew.-% der enthaltenen Tenside, bezogen auf deren Gesamtgewicht, schäumende Tenside sind. Unter einem schäumenden Tensid wird erfindungsgemäß ein Tensid verstanden, das gemäß DIN 53902 ein Schaumvermögen von mindestens 40 ml, bevorzugt von mindestens 80 ml und besonders bevorzugt mindestens 100 ml aufweist.
Erfindungsgemäß bevorzugte kationische Tenside sind ausgewählt aus Tensiden vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten der genannten Tenside weisen bevorzugt 10 bis 18 Kohlenstoffatome auf, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.
Besonders bevorzugte kationische Tenside sind Esterquats handelt es sich um bekannte Stoffe, die sowohl mindestens eine Esterfunktion als auch mindestens eine quartäre Ammoniumgruppe als Strukturelement enthalten. Erfindungsgemäß bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden zum Beispiel unter den Handelsnamen Stepantex^{®}, Dehyquart^{®} und Armocare^{®} vertrieben. Besonders bevorzugt sind Dipalmitoylethyl Hydroxyethylmonium Methosulfate, Dihydrogenated Tallowethyl Hydroxyethylmonium Methosulfate und N-Methyl-N-(2-hydroxyethyl)-N,N-di(tallow acyloxyethyl)ammonium-methosulfate, PEG-2 Dimeadowfoamamidoethylmonium Methosulfate, Dioleylethyl Hydroxyethylmonium Methosulfate, Dicocoylethyl Hydroxyethylmonium Methosulfate, Behentrimonium Methosulfate, PEG-3 Dioleylamidoethylmonium Methosulfate, N-Undecylenic Acid Propylamido-N-trimethyl Ammonium Methosulfate, Cetrimonium Methosulfate, Distearoylethyl Hydroxyethylmonium Methosulfate, Cocotrimonium Methosulfate und PEG-5 Cocomonium Methosulfate.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders bevorzugte Verbindung aus dieser Substanzgruppe stellt das unter der Bezeichnung Tegoamid^{®} S 18 im Handel erhältliche Stearamidopropyldimethylamin dar.

Besonders bevorzugte erfindungsgemäße Zusammensetzungen sind dadurch gekennzeichnet, dass sie kein kationisches Tensid und kein kationisches Polymer enthalten.

Die erfindungsgemäßen Hautreinigungszusammensetzungen enthalten, bezogen auf die Gesamtzusammensetzung, insgesamt mindestens 5 Gew.-% mindestens eines anionischen, zwitterionischen, ampholytischen, nichtionischen oder kationischen Tensids, wobei mindestens ein Tensid enthalten ist, das bei 20°C in flüssiger oder pastöser, fließfähiger Form vorliegt. Bevorzugt beträgt der gesamte Tensidgehalt 7-30 Gew.-%, besonders bevorzugt 10-15 Gew.-%, außerordentlich bevorzugt 12-14 Gew.-%, jeweils bezogen auf die Gesamtzusammensetzung.

Die Gesamtmenge an schäumendem Tensid beträgt in einer weiteren bevorzugten Ausführungsform der Erfindung 5 - 30 Gew.-%, besonders bevorzugt 3 - 25 Gew.-% und außerordentlich bevorzugt 5 - 15 Gew.-%, jeweils bezogen auf die Gesamtzusammensetzung.

Unter hydratisierenden Salzen mit negativer Lösungsenthalpie sind solche wasserlöslichen Salze zu verstehen, die sich in Wasser unter Wärmeentwicklung lösen. Dies ist in der Regel dann der Fall, wenn bei der Auflösung Hydrate gebildet werden und die Bildungswärme dieser Hydrate größer ist als die zur Überwindung der Gitterenergie verbrauchte Wärme. In der Regel handelt es sich dabei um ganz oder teilweise dehydratisierte Salze, die in Wasser Hydrate bilden. Erfindungsgemäß bevorzugte Salze dieses Typs sind z. B. Calciumchlorid, Calciumsulfat, Magnesiumchlorid, Magnesiumsulfat, Aluminiumsulfat, Chloride von Alkalimetallen, Sulfate von Alkalimetallen, z. B. Natriumchlorid oder Natriumsulfat, Carbonate und Sesquicarbonate, Ortho- und Pyrophosphate, Borate, Alkalimetallcitrate, Alkalimetallacetate, Zinkcitrat, Zinksulfat und Zinknitrat.
Erfindungsgemäß besonders bevorzugt sind die wasserlöslichen Salze, die sich in Wasser mit negativer Lösungsenthalpie lösen und nicht hydrolysieren und damit den pH-Wert des Produktes bei der Anwendung nicht beeinflussen. Die nicht-hydrolysierenden Salze sind besonders bevorzugt ausgewählt aus den wasserfreien Chloriden und Sulfaten der Alkali- und Erdalkalimetalle. Besonders bevorzugt sind Magnesiumsulfat, Natriumsulfat, Magnesiumchlorid und Calciumchlorid. Insbesondere mit diesen nicht-hydrolysierenden Salzen können pH-hautfreundliche Zusammensetzungen, die insbesondere auch zur Anwendung auf empfindlicher Haut geeignet sind, hergestellt werden.
Die erfindungsgemäßen Zusammensetzungen enthalten mindestens 5 Gew.-% mindestens eines wasserlöslichen Salzes mit negativer Lösungsenthalpie. In einer bevorzugten Ausführungsform der Erfindung ist/sind das/die wasserlösliche/n Salz/e mit negativer Lösungsenthalpie in einer Gesamtmenge von 5-20 Gew.-%, bevorzugt 7-16 Gew.-% und besonders bevorzugt 9-12 Gew.-%, jeweils bezogen auf die Gesamtzusammensetzung, enthalten.
Dabei ist darauf zu achten, dass Salze mit hoher Lösungswärme nicht in zu hoher Dosierung eingesetzt werden, damit es bei der Anwendung nicht zu einer unangenehmen Hitzeentwicklung auf der Haut kommt. Daher sollte z. B. wasserfreies MgSO₄ bevorzugt in Mengen von nicht mehr als 15 Gew.-% eingesetzt werden.

Im Stand der Technik sind zahlreiche selbsterwärmende Hautreinigungszusammensetzungen bekannt, deren Wärmeentwicklung bei Kontakt mit Wasser auf einen Gehalt an Molekularsieben oder Zeolithen zurückzuführen ist. In der vorliegenden Erfindung wurde festgestellt, dass Molekularsiebe oder Zeolithe nicht optimal zu den gewünschten Produkteigenschaften führen. Da sie nicht wasserlöslich sind, wirken sie bei der Anwendung als Peelingkörper, was zur Herstellung von Reinigungszusammensetzungen für empfindliche Haut unerwünscht sein kann. Die erfindungsgemäßen Zusammensetzungen sind daher dadurch gekennzeichnet, dass sie frei von Molekularsieben oder Zeolithen sind.

In einer weiteren bevorzugten Ausführungsform weisen die erfindungsgemäßen Zusammensetzungen bei Verdünnung zu einer 50 Gew.-%igen wässrigen Lösung bei 20°C einen pH-Wert im Bereich von 4,5 - 7,5, bevorzugt 4,9 - 7,0, besonders bevorzugt 4,9 - 5,5, auf.

In einer weiteren bevorzugten Ausführungsform liegen die erfindungsgemäßen Hautreinigungszusammensetzungen in Form einer gelförmigen, flüssigen bis hochviskosen Zubereitung vor. Die gelartige Konsistenz des erfindungsgemäßen Hautreinigungsmittels lässt sich in gewissem Ausmaß durch den Zusatz geeigneter Verdickungsmittel steuern, hängt aber letztendlich immer von Art der Gesamtzusammensetzung ab. Bevorzugt sollte die Viskosität des Produktes höher als 7 Pa·s (gemessen mit einem Brookfield Rotationsviskosimeter, Typ RTV-Helipath, Spindel T-C, bei 4 UpM (Umdrehungen pro Minute) und 20°C) liegen. Für die Anwendung der erfindungsgemäßen Hautreinigungszusammensetzung aus Tuben oder Kunststoff-Spendefläschchen ist eine Viskosität von 12 - 70 Pa·s, insbesondere von 20 - 50 Pa·s, besonders bevorzugt, außerordentlich bevorzugt ist eine Viskosität von 15 - 25 Pa·s.
Weitere bevorzugte Ausführungsformen der Erfindung sind daher dadurch gekennzeichnet, dass sie eine Viskosität von 12 - 70 Pa·s, bevorzugt 20 - 50 Pa·s, besonders bevorzugt 15 - 25 Pa.s, aufweisen, gemessen mit einem Brookfield Rotationsviskosimeter, Typ RTV mit Helipath, Spindel T-C, bei 4 UpM, 20°C.

Die erfindungsgemäßen Zusammensetzungen enthalten mindestens 0,1 Gew.-% mindestens eines organischen und/oder anorganischen Verdickungsmittels.
Bevorzugte Ausführungsformen der Erfindung sind dadurch gekennzeichnet, dass sie mindestens ein organisches Verdickungsmittel enthalten, das ausgewählt ist aus den organischen Hydrokolloiden, also natürlichen oder synthetischen Polymeren, die in Wasser quellbar oder löslich sind und die sich in der im wesentlichen aus den vorgenannten Polyolen bestehenden Trägerflüssigkeit lösen oder zumindest aufquellen lassen. Besonders bevorzugte organische Verdickungsmittel sind insbesondere nichtionische Polysaccharidderivate, wie die aus α-D-Glucose-Einheiten aufgebauten Stärken, sowie Stärkeabbauprodukte wie Amylose, Amylopektin und Dextrine. Erfindungsgemäß besonders bevorzugt sind chemisch und/oder thermisch modifizierte Stärken, z. B. Hydroxypropylstärke. Weitere erfindungsgemäß bevorzugte organische Verdickungsmittel sind nichtionische Cellulose-Derivate, wie Methylcellulose, Hydroxypropylcellulose, Hydroxypropylmethylcellulose oder Hydroxyethylcellulose. Weitere erfindungsgemäß bevorzugte organische Verdickungsmittel sind Hydroxypropylguar oder synthetische Polymere wie Polyvinylpyrrolidon, Polyvinylalkohol oder Polyacrylamid. In einer besonders bevorzugten Ausführung ist in der erfindungsgemäßen Hautreinigungszusammensetzung als Verdickungsmittel mindestens ein nichtionisches Polysaccharidderivat enthalten. Das/die organische/n Verdickungsmittel ist/sind in einer Gesamtmenge von 0,01 bis 1 Gew.-%, bevorzugt 0,1 - 0,5 Gew.-%, jeweils bezogen auf die Gesamtzusammensetzung, enthalten.

Bevorzugte Ausführungsformen der Erfindung sind dadurch gekennzeichnet, dass sie mindestens ein anorganisches Verdickungsmittel enthalten, das ausgewählt ist aus Schichtsilikaten, insbesondere Tonen (Kaolinen, Montmorilloniten, Bentoniten), die organisch modifiziert sein können (z. B. Disteardimonium Hectorite oder Quaternium-18 Hectorite), und amorphen Kieselsäuren, insbesondere pyrogenen Kieselsäuren (Aerosil) oder Fällungskieselsäuren. Das/die anorganische/n Verdickungsmittel ist/sind bevorzugt in einer Gesamtmenge von 0,1 bis 5 Gew.-%, besonders bevorzugt 0,5 - 4 Gew.-%, außerordentlich bevorzugt 1 - 3 Gew.-%, jeweils bezogen auf die Gesamtzusammensetzung, enthalten.

Bevorzugte erfindungsgemäße Hautreinigungszusammensetzungen sind weiterhin dadurch gekennzeichnet, dass sie mindestens 0,1 Gew.-% mindestens einer organischen oder anorganischen, wasserunlöslichen und mechanisch stabilen Abrasivkomponente mit einem mittleren Teilchendurchmesser von 50 - 400 µm enthalten. Als mechanisch stabil gelten erfindungsgemäß solche Abrasivkomponenten, die beim Verreiben auf der Haut nicht mechanisch zerstört werden, wie das beispielsweise bei Wirkstoff-gefüllten Mikrokapseln erwünscht wäre. Bevorzugt weist die mindestens eine organische oder anorganische Abrasivkomponente einen mittleren Teilchendurchmesser von 50 - 400 µm, bevorzugt 75 - 350 µm, besonders bevorzugt 100 - 300 µm und außerordentlich bevorzugt 150 - 300 µm, auf.
Bevorzugte erfindungsgemäße Zusammensetzungen sind dadurch gekennzeichnet, dass die mindestens eine organische, wasserunlösliche und mechanisch stabile Abrasivkomponente ausgewählt ist aus Polymerkügelchen, besonders bevorzugt aus Polyethylen oder Polyamid-11, weiterhin ausgewählt aus kristalliner Cellulose, gehärtetem Jojobaöl (Jojobabeads) und gemahlenen Pflanzenteilen wie dem gemahlenen Endocarp von Pfirsich-, Aprikosen-, Walnuss- oder Kirschkernen, dem gemahlenen, ggf. entfetteten Fruchtfleisch von Mandeln, Kokosnüssen, Jojobafrüchten, Macadamia-Nüssen und anderen Nüssen, Mandelkleie, Weizenkleie, Hafermehl, Sägemehl (Holzmehl), Nussschalenmehl, insbesondere Walnussschalenmehl, oder Maiskolbenmehl.
Weitere bevorzugte erfindungsgemäße Zusammensetzungen sind dadurch gekennzeichnet, dass die mindestens eine anorganische, wasserunlösliche und mechanisch stabile Abrasivkomponente ausgewählt ist aus Seesand, Glasstaub, Bimssteinmehl, Kreide und Muschelkalkmehl.
Weitere bevorzugte erfindungsgemäße Zusammensetzungen sind dadurch gekennzeichnet, dass die organische und/oder anorganische Abrasivkomponente oder die Mischung von organischen und anorganischen Abrasivkomponenten in einer Menge von 0,1 bis 10 Gew.-%, bevorzugt 1 - 8 Gew.-% und besonders bevorzugt 2 - 5 Gew.-%, jeweils bezogen auf die Gesamtzusammensetzung, enthalten ist.

Bevorzugte erfindungsgemäße Hautreinigungszusammensetzungen sind dadurch gekennzeichnet, dass sie mindestens einen adsorptiv wirkenden, pulverförmigen Stoff mit einer mittleren Teilchengröße kleiner 50 µm enthalten. Solche Stoffe, die sich durch eine große Oberfläche auszeichnen, können anorganische Pulver, besonders bevorzugt Talkum, daneben aber auch Veegum^{®} (synthetische Magnesiumaluminiumsilikate), Aluminiumoxide oder nanopartikuläre Salze sein. Es kann sich aber auch um organische, feinteilige Adsorbentien wie z. B. Cellulose, modifizierte Stärke oder Polymerpulver, wie Polyamidpulver, mit einer mittleren Teilchengröße kleiner 50 µm handeln. Ein besonders bevorzugtes teilchenförmiges Adsorptionsmittel ist Talkum. In einer bevorzugten Ausführungsform enthalten die erfindungsgemäßen Hautreinigungszusammensetzungen mindestens ein teilchenförmiges, anorganisches oder organisches Adsorptionsmittels mit einer mittleren Teilchengröße kleiner 50 µm, besonders bevorzugt Talkum, in einer Gesamtmenge von 3 - 30 Gew.-%, bevorzugt 5 - 20 Gew.-%, besonders bevorzugt 10 - 17 Gew.-% und außerordentlich bevorzugt 13 - 15 Gew.-%, jeweils bezogen auf die gesamte Zusammensetzung.

Zusätzlich zu den genannten Komponenten können in dem erfindungsgemäßen Reinigungsmittel weitere in Körperreinigungsmitteln übliche Hilfsmittel und Zusätze enthalten sein. Solche Zusätze dienen der Verbesserung der Hautverträglichkeit und der kosmetischen Sensorik ganz allgemein. Geeignete Stoffe sind z. B. Feuchthaltemittel wie z. B. Pyrrolidoncarbonsäure, keratolytische und hautweichmachende Komponenten wie z. B. Harnstoff, Allantoin, rückfettende Komponenten wie z. B. Lipidkomponenten und Silikone, Fette und Wachse, insbesondere solche, die in mikroemulgierter (in der hydrophilen wasserfreien Phase) oder nanopartikulärer Form vorliegen, Vitamine wie Tocopherol, Retinol oder Ascorbinsäure, Panthenol oder Biotin sowie Vitaminderivate, insbesondere Tocopherolacetat, Retinylpalmitat oder Pantolacton, wasserlösliche Proteinderivate, Duftstoffe, Farbstoffe und Perlglanzpigmente.

Weitere Hilfsmittel, die vorwiegend der Lagerstabilität dienen, sind z. B. Konservierungsstoffe, Komplexbildner, pH-Regulatoren und Puffersubstanzen.

Die Herstellung der erfindungsgemäßen Hautreinigungszusammensetzungen erfolgt durch Vermischen der Komponenten unter Erwärmen. Dabei legt man die flüssigen Trägerkomponenten, also die wasserlöslichen Polyole, vor und löst oder dispergiert darin die Verdickungsmittel, dann fügt man nacheinander die Tenside, die Adsorbentien und zuletzt die hydratisierenden Salze zu. Es empfiehlt sich, dabei in einer geschlossenen Anlage unter leichtem Unterdruck zu arbeiten, damit im Produkt keine Luft eingeschlossen wird und nicht zuviel Luftfeuchtigkeit zutreten kann.

Ein weiterer Gegenstand der vorliegenden Erfindung ist die Verwendung einer erfindungsgemäßen Hautreinigungszusammensetzung gemäß einem der Patentansprüche 1 - 16 zur Make-up-Entfernung und/oder als Duschgel.

Ein weiterer Gegenstand der vorliegenden Erfindung ist die Verwendung einer erfindungsgemäßen Hautreinigungszusammensetzung gemäß einem der Patentansprüche 1 - 16 zur Ausrüstung von flexiblen, wasserunlöslichen Trägern, insbesondere ein- oder mehrlagigen Tüchern aus Geweben oder nicht-gewebten Vliesen sowie Schwämmen.
Ein weiterer Gegenstand der vorliegenden Erfindung ist die Verwendung einer erfindungsgemäßen Hautreinigungszusammensetzung gemäß einem der Patentansprüche 1-16 zur Ausrüstung von flexiblen, wasserunlöslichen Trägern, insbesondere ein- oder mehrlagigen Tüchern aus Geweben oder nicht-gewebten Vliesen sowie Schwämmen, wobei die flexiblen, wasserunlöslichen Träger dadurch gekennzeichnet sind, dass sie trocken sind. Unter "trocken" wird erfindungsgemäß ein Wassergehalt von maximal 10 Gew.-%, bevorzugt maximal 8 Gew.-%, besonders bevorzugt 5 Gew.-%, jeweils bezogen auf das imprägnierte, getrocknete Tuch, verstanden.
Ein weiterer Gegenstand der vorliegenden Erfindung ist ein Verfahren zur Reinigung der Haut, das dadurch gekennzeichnet ist, dass ein flexibler, wasserunlöslicher Träger, der mit einer Hautreinigungszusammensetzung gemäß einem der Ansprüche 1 - 16 ausgerüstet ist, mit Wasser angefeuchtet und mit der Haut in Kontakt gebracht wird.

Geeignete und bevorzugte Verpackungsbehälter für die erfindungsgemäßen Hautreinigungszusammensetzungen können vorteilhaft aus einer Tube oder aus einer Kunststoffflasche mit einem Pumpspenderaufsatz bestehen. Vorteilhafterweise kann die erfindungsgemäße Hautreinigungszusammensetzung auch in einer Aerosoldose verpackt sein und mit einem Treibmittel aufgeschäumt werden.

Die folgenden Beispiele sollen den Gegenstand der Erfindung näher erläutern, ohne ihn hierauf zu beschränken.

Es wurden folgende Beispielzusammensetzungen hergestellt (Angaben in Gew.-%)

| | Nr.1 | Nr. 2 | Nr. 3 | Nr. 4 | Nr. 5 | Nr. 6 |
|---|---|---|---|---|---|---|
| Polyethylenglycol 400 | 30 | 30 | 30 | 30 | 30 | 30 |
| 1,2-Propylenglycol | 11,5 | 11,5 | 11,5 | 11,5 | 11,5 | 11,5 |
| Sorbit (100 %, Pulver) | 3,0 | 3,0 | 3,0 | 3,0 | 3,0 | 3,0 |
| Glycerin (99,5 %ig) | 5,0 | 5,0 | 5,0 | 5,0 | 5,0 | 5,0 |
| Dow Corning^{®} 193 | - | - | - | - | 1,0 | 1,0 |
| Polysorbate 20 | 1,5 | 1,5 | 1,5 | 1,5 | 1,5 | 1,5 |
| Elfan^{®} AT 84 | 8,0 | - | - | - | - | - |
| Sulfosuccinat 128 P | 5,0 | - | - | - | - | - |
| C10-C16-Alkylglucosid | - | - | - | - | 6,5 | - |
| Dehydol LT 7 | - | - | - | - | 6,5 | - |
| Marlinat 242/90 M | - | - | 6,5 | 13,0 | - | 13,0 |
| Plantapon LC 7 | - | - | 6,5 | - | - | - |
| Glucopon anhydrous | - | 13,0 | - | - | - | - |
| Parfümöl | 0,3 | 0,3 | 0,3 | 0,3 | 0,3 | 0,5 |
| Tocopherylacetat | 0,5 | 0,5 | 0,5 | 0,5 | 0,5 | 0,5 |
| Ginseng-Extrakt, öllöslich (Isopropylmyristat) | 1,0 | 1,0 | 1,0 | 1,0 | - | 1,0 |
| Bisabolol | - | - | - | - | 1,0 | 1,0 |
| Allantoin | - | - | - | - | - | 1,0 |
| Lipochroman-6 | - | - | - | - | - | 0,8 |
| Retinylpalmitat | - | - | - | - | - | 1,0 |
| Talkum | 12 | 12 | 12 | 12 | 11 | 12 |
| Aerosil^{®} 200 | 4,0 | 4,0 | 4,0 | 4,0 | 4,0 | 4,0 |
| MgSO₄ (wasserfrei) | 13 | 13 | 13 | 13 | 13 | 13 |
| Microscrub 50 PC | 5 | 5 | 5 | 5 | 5 | - |
| TiO₂ | 0,2 | 0,2 | 0,2 | 0,2 | 0,2 | 0,2 |
| Viskosität (Pa·s) (Brookfield, Type RTV, Spindel T-C, 4 UpM, 20°C) | 20 - 25 | 50 - 70 | 12 - 15 | 8 - 10 | 50 - 70 | 7 - 9 |
| pH-Wert in 50 Gew.-%iger wässriger Lösung (20°C) | 5,1 - 5,4 | 5,1 - 5,4 | 5,1 - 5,4 | 5,1 - 5,4 | 5,1 - 5,4 | 5,1 - 5,4 |

Die nicht erfindungsgemäße Rezeptur Nr. 1 zeigte bei Raumtemperatur eine hohe Lagerstabilität und eine gute Konsistenz bzw. Viskosität zur Dosierung aus einer Kopfstandtube, allerdings verdickte die Zusammensetzung bei längerer Lagerung bei 40 °C so stark, dass sie nicht mehr aus der Tube zu dosieren war. Die erfindungsgemäßen Rezepturen 2 - 6 waren auch bei 40°C lagerstabil und zeigten lediglich Änderungen in der Viskosität von maximal ± 15 % der Anfangsviskosität bei 20 °C.

Es wurden folgende Handelsprodukte verwendet:
- Elfan^{®} AT 84 (Akzo Nobel):: Kokosacylisethionat, Na-Salz (Pulver mit 84 Gew.-% Aktivsubstanz)
- Sulfosuccinat 128 P (Cognis):: Sulfobernsteinsäure-mono(C₁₂₋₁₈)-alkylester, Di-Na-Salz (Pulver, 90 % Aktivsubstanz)
- Aerosil^{®} 200 (Degussa):: Kieselsäure, pyrogen, amorph
- Polyethylenglycol 400: PEG-8
- Dow Corning^{®} 193:: PEG-12 Dimethicone
- Microscrub 50 PC (Micro Powders Inc.): Polyethylene
- C10-C16-Alkylglucosid: ex Cognis
- Dehydol LT 7 (Cognis): Laureth-7
- Marlinat 242/90 M (Sasol): MIPA Laureth-2 sulfate (82 - 88 Gew.-%), Laureth-4 (3-5 Gew.-%), Propylenglycol (10-12 Gew.-%) (Viskosität: 1500 mPa·s bei 20°C, gemessen nach DIN ISO 2555)
- Plantapon LC 7: Laureth-7 Citrate
- Glucopon anhydrous (Cognis): C10-C16-Alkylglucosid, Laureth-7
- Lipochroman-6 (Lipotec): Dimethylmethoxy chromanol

## Patentansprüche

1. Wasserfreie, selbsterwärmende Hautreinigungszusammensetzung, die beim Vermischen mit Wasser Hydratationswarme erzeugt, enthaltend (jeweils bezogen auf die Gesamtzusammensetzung):
a) mindestens 15 Gew.-% mindestens eines wasserlöslichen mehrwertigen C₂-C₉-Alkanols mit 2 - 6 Hydroxylgruppen und/oder mindestens eines wasserlöslichen Polyethylenglycols mit 3-20 Ethylenoxid-Einheiten und/oder mindestens eines wasserlöslichen Polypropylenglycols und/oder mindestens eines wasserlöslichen Anlagerungsproduktes von Ethylenoxid an Propylenglycol oder an Polypropylenglycol sowie Mischungen dieser Komponenten,
b) mindestens 5 Gew.-% mindestens eines anionischen, zwitterionischen, ampholytischen, nichtionischen oder kationischen Tensids oder Mischungen hiervon,
c) mindestens 5 Gew.-% mindestens eines wasserlöslichen Salzes, das sich in Wasser mit negativer Lösungsenthalpie löst sowie
d) mindestens 0,1 Gew.-% mindestens eines organischen und/oder anorganischen Verdickungsmittels,
**dadurch gekennzeichnet, dass** mindestens 50 Gew.%, bevorzugt mindestens 80 Gew.-% und besonders bevorzugt 100 Gew.-% der enthaltenen Tenside bei 20°C in flüssiger oder, bevorzugt, pastöser, fließfähiger Form vorliegen (Anteilsangaben bezogen auf das Gesamtgewicht der enthaltenen Tenside) und dass die Zusammensetzung frei von Molekularsieben oder Zeolithen ist.

2. Hautreinigungszusammensetzung gemäß Anspruch 1, **dadurch gekennzeichnet, dass** das/die wasserlösliche/n Salz/e, das/die sich in Wasser mit negativer Lösungsenthalpie löst/lösen, nicht hydrolysiertlen.

3. Hautreinigungszusammensetzung gemäß Anspruch 2, **dadurch gekennzeichnet, dass** das/die wasserlösliche/n Salz/e, das/die sich in Wasser mit negativer Lösungsenthalpie löst/lösen und nicht hydrolysiert/en, ausgewählt ist/sind aus den wasserfreien Chloriden und Sulfaten der Alkali- und Erdalkalimetalle.

4. Hautreinigungszusammensetzung gemäß einem der vorstehenden Ansprüche, **dadurch gekennzeichnet, dass** das/die wasserlösliche/n Salz/e in einer Menge von 5 - 20 Gew.-%, bevorzugt 7-16 Gew.-% und besonders bevorzugt 9-12 Gew.-%, jeweils bezogen auf die Gesamtzusammensetzung, enthalten sind.

5. Hautreinigungszusammensetzung gemäß einem der vorstehenden Ansprüche, **dadurch gekennzeichnet, dass** die wasserlöslichen mehrwertigen C₂ - C₈-Alkanole mit 2 - 6 Hydroxylgruppen und/oder die wasserlöslichen Polyethylenglycole mit 3 - 20 Ethylenoxid-Einheiten und/oder die wasserlöslichen Polypropylenglycole und/ oder die wasserlöslichen Anlagerungsprodukte von Ethylenoxid an Propylenglycol oder an Polypropylenglycol (Komponente a) in einer Gesamtmenge von mindestens 20 Gew.-%, bevorzugt 30 - 70 Gew.-% und besonders bevorzugt 40 - 60 Gew.-%, bezogen auf die Gesamtzusammensetzung, enthalten sind.

6. Hautreinigungszusammensetzung gemäß einem der vorstehenden Ansprüche, **dadurch gekennzeichnet, dass** das mindestens eine wasserfreie Tensid, das bei 20°C in flüssiger oder pastöser, fließfähiger Form vorliegt, ausgewählt ist aus Alkylpolyglycolethersulfaten der Formel R-O(CH₂-CH₂O)ₓ-OSO₃H, in der R eine bevorzugt lineare Alkylgruppe mit 8 bis 30 C-Atomen und x = 0 oder 1-12, bevorzugt 1-4, ist, Citronensäurealkylpolyglycolethermonoestern, Citronensäurealkylpolyglycoletherdiestern, Weinsäurealkylpolyglycolethermonoestern, Weinsäurealkylpolyglycoletherdiestern, Alkylsulfaten, Alkansulfonaten, Alpha-Olefinsulfonaten, Acylisethionaten, Acyltauriden, Acylsarkosiden, Sulfobernsteinsäuremonoalkylester-Salzen und Alkylpolyglycolethercarboxylaten in Form ihrer Alkalimetallsalze, Ammoniumsalze oder Mono-, Di- oder/und Trialkanolammoniumsalze, besonders bevorzugt in Form ihrer Mono-, Di- oder/und Trialkanolammoniumsalze, außerordentlich bevorzugt in Form ihrer Monoalkanolammoniumsalze, insbesondere in Form ihrer Monoisoalkanolammoniumsalze, wie z. B. die Monoisopropanolammoniumsalze oder Monoisobutanolammoniumsalze, weiterhin aus Alkyl- und Alkenyloligoglucosiden mit einer durchschnittlichen Alkyl- oder Alkenylkettenlänge von 8 - 12 Kohlenstoffatomen, Anlagerungsprodukten von 1-10 Ethylenoxideinheiten an gesättigte, lineare und verzweigte Alkanole mit 8 - 20 Kohlenstoffatomen, sowie Mischungen der vorgenannten Substanzen.

7. Hautreinigungszusammensetzung gemäß einem der vorstehenden Ansprüche, **dadurch gekennzeichnet, dass** das mindestens eine Tensid in einer Gesamtmenge von 7 - 30 Gew.%, bevorzugt 10-15 Gew.% und besonders bevorzugt 12 -14 Gew.-%, bezogen auf die Gesamtzusammensetzung, enthalten ist.

8. Hautreinigungszusammensetzung gemäß einem der vorstehenden Ansprüche, **dadurch gekennzeichnet, dass** sie mindestens einen adsorptiv wirkenden, pulverförmigen Stoff mit einer mittleren Teilchengröße kleiner 50 µm, insbesondere Talkum, enthalten.

9. Hautreinigungszusammensetzung gemäß Anspruch 9, **dadurch gekennzeichnet, dass** das mindestens eine teilchenförmige, anorganische oder organische Adsorptionsmittel mit einer mittleren Teilchengröße kleiner 50 µm, besonders bevorzugt Talkum, in einer Gesamtmenge von 3-30 Gew.-%, bevorzugt 5-20 Gew.-%, besonders bevorzugt 10-17 Gew.-% und außerordentlich bevorzugt 13 - 15 Gew.-%, jeweils bezogen auf die gesamte Zusammensetzung, enthalten ist.

10. Hautreinigungszusammensetzung gemäß einem der vorstehenden Ansprüche, **dadurch gekennzeichnet, dass** weiterhin mindestens 0,1 Gew.-% mindestens einer organischen oder anorganischen, wasserunlöslichen und mechanisch stabilen Abrasivkomponente mit einem mittleren Teilchendurchmesser von 50 - 400 µm enthalten ist.

11. Hautreinigungszusammensetzung gemäß Anspruch 11, **dadurch gekennzeichnet, dass** die organische, wasserunlösliche und mechanisch stabile Abrasivkomponente mit einem mittleren Teilchendurchmesser von 50 - 400 µm ausgewählt ist aus Polymerkügelchen, bevorzugt aus Polyethylen oder Polyamid-11, kristalliner Cellulose, gehärtetem Jojobaöl (Jojobabeads) und gemahlenen Pflanzenteilen wie dem gemahlenen Endocarp von Pfirsich-, Aprikosen-, Walnuss- oder Kirschkernen, dem gemahlenen, ggf. entfetteten Fruchtfleisch von Mandeln, Kokosnüssen, Jojobafrüchten, Macadamia-Nüssen und anderen Nüssen, Mandelkleie, Weizenkleie, Hafermehl, Sägemehl (Holzmehl), Nussschalenmehl oder Maiskolbenmehl, sowie Mischungen dieser Komponenten.

12. Hautreinigungszusammensetzung gemäß Anspruch 11, **dadurch gekennzeichnet, dass** die anorganische, wasserunlösliche und mechanisch stabile Abrasivkomponente mit einem mittleren Teilchendurchmesser von 60 - 400 µm ausgewählt ist aus Seesand, Glasstaub, Bimssteinmehl, Kreide und Muschelkalkmehl.

13. Hautrelnigungszusammensetzung gemäß einem der vorstehenden Ansprüche, **dadurch gekennzeichnet, dass** kein kationisches Tensid und kein kationisches Polymer enthalten ist.

14. Hautreinigungszusammensetzung gemäß einem der vorstehenden Ansprüche, **dadurch gekennzeichnet, dass** die Zusammensetzung In einer 50 Gew.-%igen wässrigen Lösung bei 20°C einen pH-Wert im Bereich von 4,5 - 7,5, bevorzugt 4,9 - 7,0, besonders bevorzugt 4,9 - 5,5, aufweist.

15. Hautreinigungszusammensetzung gemäß einem der vorstehenden Ansprüche, **gekennzeichnet durch** eine Viskosität von 12 - 70 Pa·s, bevorzugt 20 - 50 Pa·s, besonders bevorzugt 15 - 25 Pa·s, jeweils gemessen mit einem Brookfield Rotationsviskosimeter, Typ RTV-Helipath, Spindel TC, bei 4 Umdrehungen pro Minute und 20°C.

16. Verwendung einer Hautreinigungszusammensetzung gemäß einem der Ansprüche 1 -15 zur Make-up-Entfernung und/oder als Duschgel.

17. Verwendung einer Hautreinigungszusammensetzung gemäß einem der Ansprüche 1 -15 zur Ausrüstung flexibler, wasserunlöslicher Träger, insbesondere eln- oder mehrlagiger Tücher aus Geweben oder nicht-gewebten Vliesen sowie Schwämmen.

18. Verwendung gemäß Anspruch 17, **dadurch gekennzeichnet, dass** der flexible, wasserunlösliche Träger trocken ist.

19. Verfahren zur Reinigung der Haut, **dadurch gekennzeichnet, dass** ein flexibler, wasserunlöslicher Träger, der mit einer Hautreinigungszusammensetzung gemäß einem der Ansprüche 1 - 16 ausgerüstet ist, mit Wasser angefeuchtet und mit der Haut In Kontakt gebracht wird.

20. Hautreinigungszusammensetzung gemäß einem der Ansprüche 1-15, **dadurch gekennzeichnet, dass** sie in einer Aerosoldose verpackt ist und mit einem Treibmittel aufgeschäumt werden kann.

## Claims

1. An anhydrous self-heating composition for cleansing the skin, which, when it is mixed with water, produces hydration heat, containing (each based on the total composition):
a) at least 15% by weight of at least a water-soluble multivalent C₂ -C₉ alkanol with 2-6 hydroxyl groups and/or of at least one water-soluble polyethylene glycol having 3-20 ethylene oxide units and/or of at least one water-soluble polypropylene glycol and/or of at least a water-soluble addition product of ethylene oxide on propylene glycol or on polypropylene glycol as well as mixtures of these components,
b) at least 5% by weight of at least one anionic, zwitterionic, ampholytic, non-ionic or cationic surfactant or mixtures thereof,
c) at least 5% by weight of at least one water-soluble salt which dissolves in water with a negative dissolution enthalpy as well as
d) at least 0.1% by weight of at least an organic and/or inorganic thickener,
**characterized in that** at least 50% by weight, preferably at least 80% by weight and more preferably 100% by weight of the contained surfactants exist at 20°C in a liquid or preferably pasty, flowable form (content indications are based on the total weight of the contained surfactants) and **in that** the composition is free of molecular sieves or zeolites.

2. The skin cleansing composition according to claim 1, **characterized in that** the water-soluble salt(s) which dissolve in water with a negative dissolution enthalpy, are not hydrolyzed.

3. The skin cleansing composition according to claim 2, **characterized in that** the water-soluble salt(s) which dissolves in water with a negative dissolution enthalpy and which are not hydrolyzed, are selected from anhydrous chlorides and sulfates of alkaline and earth alkaline metals.

4. The skin cleansing composition according to any of the preceding claims, **characterized in that** the water-soluble salt(s) are contained in an amount from 5 to 20% by weight, preferably from 7 to 16% by weight, and more preferably from 9 to 12% by weight, each based on the total composition.

5. The skin cleansing composition according to any of the preceding claims, **characterized in that** the water-soluble multivalent C₂-C₉ alkanols with 2-6 hydroxyl groups, and/or the water-soluble polyethylene glycols having 3-20 ethylene oxide units and/or the water-soluble polypropylene glycol and/or the water-soluble addition products of ethylene oxide on propylene glycol or on polypropylene glycol (component a) are contained in a total amount of at least 20% by weight, preferably from 30 to 70% by weight, and more preferably from 40 to 60% by weight, based on the total composition.

6. The skin cleansing composition according to any of the preceding claims, **characterized in that** said at least one anhydrous surfactant which exists at 20°C in a flowable, liquid or pasty form, is selected from alkylpolyglycolether sulfates of formula R-O(CH₂-CH₂O)ₓ-OSO₃H, wherein R is a preferably linear alkyl group with 8-30 C atoms and x = 0 or 1-12, preferably 1-4, alkylpolyglycolether citric acid monoesters, alkylpolyglycolether citric acid diesters, alkylpolyglycolether tartaric acid monoesters, alkylpolyglycolether tartaric acid diesters, alkyl sulfates, alkane-sulfonates, alpha-olefin sulfonates, acyl isethionate, acyl taurides, acyl sarcosides, sulfosuccinic acid monoalkylester salts, alkylpolyglycolether carboxylates in the form of their alkaline metal salts, ammonium salts, or mono-, di- or/and tri-alkanolammonium salts, more preferably in the form of their mono-, di- and/or tri-alkanolammonium salts, extremely preferably in the form of their monoalkanolammonium salts, in particular in the form of their monoisoalkanolammonium salts, such as for example monoisopropanol ammonium salts or monoisobutanol ammonium salts, further from alkyl and alkenyl oligoglucosides, with an average alkyl or alkenyl chain length of 8-12 carbon atoms, addition products of 1-10 ethylene oxide units on linear and branched saturated alkanols, with 8-20 carbon atoms as well as the mixtures of the aforementioned substances.

7. The skin cleansing composition according to any of the preceding claims, **characterized in that** said at least one surfactant is contained in a total amount from 7 to 30% by weight, preferably from 10 to 15% by weight, and more preferably from 12 to 14% by weight, based on the total composition.

8. The skin cleansing composition according to any of the preceding claims, **characterized in that** it contains at least one powdery substance having an adsorption effect with an average particle size smaller than 50 µm, in particular talc.

9. The skin cleansing composition according to claim 9, **characterized in that** said at least one inorganic or organic adsorption agent has particles with an average particle size smaller than 50 µm, more preferably talc, is contained in a total amount of 3-30% by weight, preferably 5-20% by weight, more preferably 10-17% by weight, and extremely preferably 13-15% by weight, each based on the total composition.

10. The skin cleansing composition according to any of the preceding claims, **characterized in that** further at least 0.1% by weight of at least an organic or inorganic abrasive component, insoluble in water and mechanically stable, with an average particle diameter of 50-400 µm is contained.

11. The skin cleansing composition according to claim 11, **characterized in that** the organic abrasive component, insoluble in water and mechanically stable, with an average particle diameter of 50-400 µm, is selected from polymer beads, preferably polyethylene or polyamide 11, crystalline cellulose, hardened jojoba oil (jojoba beads), and milled plant portions such as the milled endocarp of peach, apricot, walnut or cherry kernels, milled, optionally degreased, fruit pulp of almonds, coconuts, jojoba fruit; macadamia nuts and other nuts, almond bran, wheat brand, oats flour, sawdust (wood meal), nutshell meal or maize cob meal, as well as mixtures of these components.

12. The skin cleansing composition according to claim 11, **characterized in that** the inorganic abrasive component, insoluble in water and mechanically stable, with an average particle diameter of 50-400 µm, is selected from beach sand, glass dust, pumice stone meal, chalk and ground shell marl.

13. The skin cleansing composition according to any of the preceding claims, **characterized in that** no cationic surfactant and no cationic polymer are contained.

14. The skin cleansing composition according to any of the preceding claims, **characterized in that** the composition has a pH value in the range from 4.5 to 7.5, preferably from 4.9 to 7.0, more preferably from 4.9 to 5.5, in a 50% by weight aqueous solution, at 20°C.

15. The skin cleansing composition according to any of the preceding claims, **characterized by** a viscosity of 12-70 Pa.s, preferably 20-50 Pa.s, more preferably 15-25 Pa.s, each measured with a rotary Brookfield viscosimeter of the RTV Helipath type, spindle TC, 4 revolutions per minute, and at 20°C.

16. The use of a skin cleansing composition according to any claims 1 to 15 for removing make-up and/or as a shower gel.

17. The use of a skin cleansing composition according to any of claims 1 to 15 for finishing flexible, water-insoluble carriers, in particular monolayer or multilayer tissues consisting of woven or non-woven tissues as well as sponges.

18. The use according to claim 17, **characterized in that** the flexible water-insoluble carrier is dry.

19. A skin cleansing method, **characterized in that** a flexible water-insoluble carrier which is finished with a skin cleansing composition according to any of claims 1 to 15, is moistened with water and brought into contact with the skin.

20. The skin cleansing composition according to any of claims 1 to 15, **characterized in that** it is packaged in an aerosol spray and may be foamed with a propellant.

## Revendications

1. Composition auto-chauffante anhydre pour le nettoyage de la peau, qui, lorsqu'elle est mélangée à de l'eau produit une chaleur d'hydratation, contenant (à chaque fois par rapport à la composition totale) :
a) au moins 15% en poids d'au moins un alcanol polyvalent en C₂ à C₉, hydrosoluble, ayant de 2 à 6 groupes hydroxyle et/ou d'au moins un polyéthylèneglycol hydrosoluble ayant de 3 à 20 motifs oxyde d'éthylène et/ou d'au moins un polypropylèneglycol hydrosoluble et/ou d'au moins un produit hydrosoluble d'addition d'oxyde d'éthylène sur du propylèneglycol ou sur du polypropylèneglycol ainsi que de mélanges de ces composants,
b) au moins 5% en poids d'au moins un tensio-actif anionique, zwitterionique, ampholytique, non-ionique ou cationique, ou des mélanges de ceux-ci,
c) au moins 5% en poids d'au moins un sel hydrosoluble, qui se dissout dans de l'eau avec une enthalpie de dissolution négative ainsi que
d) au moins 0,1% en poids d'au moins un épaississant organique et/ou inorganique,
**caractérisée en ce qu'**au moins 50% en poids, de préférence au moins 80% en poids et de manière particulièrement préférée 100% en poids des tensio-actifs contenus existent à 20°C sous une forme liquide ou de préférence pâteuse, fluide (indications des teneurs rapportées au poids total des tensio-actifs contenus) et que la composition est exempte de tamis moléculaires ou de zéolithes.

2. Composition pour le nettoyage de la peau selon la revendication 1, **caractérisée en ce que** le ou les sels hydrosolubles qui se dissolvent dans de l'eau avec une enthalpie de dissolution négative, ne sont pas hydrolysés.

3. Composition pour le nettoyage de la peau selon la revendication 2, **caractérisée en ce que** le ou les sels hydrosolubles qui se dissolvent dans de l'eau avec une enthalpie de dissolution négative et qui ne sont pas hydrolysés, sont choisis parmi les chlorures et sulfates anhydres des métaux alcalins et alcalino-terreux.

4. Composition pour le nettoyage de la peau selon l'une quelconque des revendications précédentes, **caractérisée en ce que** le ou les sels hydrosolubles sont contenus en une quantité de 5 à 20% en poids, de préférence de 7 à 16% en poids, et de manière particulièrement préférée de 9 à 12% en poids, à chaque fois par rapport à la composition totale.

5. Composition pour le nettoyage de la peau selon l'une quelconque des revendications précédentes, **caractérisée en ce que** les alcanols en C₂ à C₉ polyvalents, hydrosolubles, ayant de 2 à 6 groupes hydroxyle, et/ou les polyéthylèneglycols hydrosolubles ayant de 3 à 20 motifs oxyde d'éthylène et/ou les polypropylèneglycols hydrosolubles et/ou les produits hydrosolubles d'addition de l'oxyde d'éthylène sur le propylèneglycol ou sur le polypropylèneglycol (composant a) sont contenus en une quantité totale d'au moins 20% en poids, de préférence de 30 à 70% en poids, et de manière particulièrement préférée de 40 à 60% en poids, par rapport à la composition totale.

6. Composition pour le nettoyage de la peau selon l'une quelconque des revendications précédentes, **caractérisée en ce que** ledit au moins un tensio-actif anhydre qui existe à 20°C sous une forme fluide, liquide ou pâteuse, est choisi parmi les alkylpolyglycoléther-sulfates de formule R-O(CH₂-CH₂O)ₓ-OSO₃H, dans laquelle R est un groupe alkyle de préférence linéaire ayant de 8 à 30 atomes de C et x = 0 ou vaut de 1 à 12, de préférence de 1 à 4, les monoesters d'alkylpolyglycoléther avec l'acide citrique, les diesters d'alkylpolyglycoléther avec l'acide citrique, les monoesters d'alkylpolyglycoléther avec l'acide tartrique, les diesters d'alkylpolyglycoléther avec l'acide tartrique, les sulfates d'alkyle, les alcane-sulfonates, les sulfonates d'alpha-oléfine, l'iséthionate d'acyle, les taurides d'acyle, les sarcosides d'acyle, les sels monoalkylesters d'acide sulfosuccinique, et les carboxylates d'alkylpolyglycoléther sous forme de leurs sels de métal alcalin, sels d'ammonium ou sels de mono-, di- ou/et tri-alcanolammonium, de manière particulièrement préférée sous forme de leurs sels de mono-, di- et/ou tri-alcanolammonium, de manière extrêmement préférée sous forme de leurs sels de monoalcanolammonium, en particulier sous forme de leurs sels de monoisoalcanolammonium, comme par exemple les sels de monoisopropanolammonium ou les sels de monoisobutanolammonium, en outre parmi les oligoglucosides d'alkyle et d'alcényle, avec une longueur de chaîne alkyle ou alcényle moyenne de 8 à 12 atomes de carbone, les produits d'addition de 1 à 10 motifs d'oxyde d'éthylène sur des alcanols saturés, linéaires et ramifiés, ayant de 8 à 20 atomes de carbone, ainsi que les mélanges des substances précitées.

7. Composition pour le nettoyage de la peau selon l'une quelconque des revendications précédentes, **caractérisée en ce que** ledit au moins un tensio-actif est contenu en une quantité totale de 7 à 30% en poids, de préférence de 10 à 15% en poids, et de manière particulièrement préférée de 12 à 14% en poids, par rapport à la composition totale.

8. Composition pour le nettoyage de la peau selon l'une quelconque des revendications précédentes, **caractérisée en ce qu'**elle contient au moins une substance pulvérulente à effet d'adsorption ayant une taille de particule moyenne inférieure à 50 µm, en particulier le talc.

9. Composition pour le nettoyage de la peau selon la revendication 9, **caractérisée en ce que** ledit au moins un agent d'adsorption inorganique ou organique sous forme de particules ayant une taille moyenne de particule inférieure à 50 µm, de manière particulièrement préférée le talc, est contenu en une quantité totale de 3 à 30% en poids, de préférence 5 à 20% en poids, de manière particulièrement préférée de 10 à 17% en poids, et de manière extrêmement préférée de 13 à 15% en poids, à chaque fois par rapport à la composition totale.

10. Composition pour le nettoyage de la peau selon l'une quelconque des revendications précédentes, **caractérisée en ce que**, en outre, au moins 0,1% en poids d'au moins un composant abrasif organique ou inorganique, insoluble dans l'eau et mécaniquement stable, ayant un diamètre moyen de particule de 50 à 400 µm est contenu.

11. Composition pour le nettoyage de la peau selon la revendication 11, **caractérisée en ce que** le composant abrasif organique, insoluble dans l'eau et mécaniquement stable, ayant un diamètre moyen de particule de 50 à 400 µm est choisi parmi les perles de polymère, de préférence le polyéthylène ou le polyamide 11, la cellulose cristalline, l'huile de jojoba durcie (perles de jojoba), et les parties végétales broyées comme l'endocarpe broyé de noyaux de pêche, d'abricot, de noix ou de cerise, la pulpe de fruit broyée, éventuellement dégraissée d'amandes, de noix de coco, de fruits du jojoba; de noix de macadamia et d'autres noix, le son d'amandes, le son de blé, la farine d'avoine, la sciure (farine de bois), la farine de coques de noix ou la farine d'épis de maïs, ainsi que les mélanges de ces composants.

12. Composition pour le nettoyage de la peau selon la revendication 11, **caractérisée en ce que** le composant abrasif inorganique, insoluble dans l'eau et mécaniquement stable, ayant un diamètre moyen de particule de 50 à 400 µm, est choisi parmi le sable marin, la poussière de verre, la pierre ponce pulvérisée, la craie, et le calcaire coquillier pulvérisé.

13. Composition pour le nettoyage de la peau selon l'une quelconque des revendications précédentes, **caractérisée en ce qu'**aucun tensio-actif cationique et aucun polymère cationique ne sont contenus.

14. Composition pour le nettoyage de la peau selon l'une quelconque des revendications précédentes, **caractérisée en ce que** la composition présente une valeur de pH dans la plage de 4,5 à 7,5, de préférence de 4,9 à 7,0, de manière particulièrement préférée de 4,9 à 5,5, dans une solution aqueuse à 50% en poids, à 20°C.

15. Composition pour le nettoyage de la peau selon l'une quelconque des revendications précédentes, **caractérisée par** une viscosité de 12 à 70 Pa.s, de préférence 20 à 50 Pa.s, de manière particulièrement préférée de 15 à 25 Pa.s, mesurée à chaque fois avec un viscosimètre rotatif Brookfield du type RTV Helipath, broche TC, à 4 tours par minute, et à 20°C.

16. Utilisation d'une composition pour le nettoyage de la peau selon l'une quelconque des revendications 1 à 15 pour retirer le maquillage et/ou en tant que gel de douche.

17. Utilisation d'une composition pour le nettoyage de la peau selon l'une quelconque des revendications 1 à 15 pour apprêter des supports souples insolubles dans l'eau, en particulier des tissus monocouche ou multicouche constitués de tissu tissé ou de non tissé, ainsi que les éponges.

18. Utilisation selon la revendication 17, **caractérisée en ce que** le support souple insoluble dans l'eau est sec.

19. Procédé pour nettoyer la peau, **caractérisé en ce qu'**un support souple insoluble dans l'eau qui est apprêté avec une composition pour le nettoyage de la peau selon l'une quelconque des revendications 1 à 15, est humidifié avec de l'eau et amené en contact avec la peau.

20. Composition pour le nettoyage de la peau selon l'une quelconque des revendications 1 à 15, **caractérisée en ce qu'**elle est conditionnée dans une bombe aérosol et que l'on peut la faire mousser avec un agent propulseur.
